# EUROPÄISCHE PATENTANMELDUNG

(11) **EP 0 866 064 A1**
(43) Veröffentlichungstag der Anmeldung: **23.09.1998**
(21) Anmeldenummer: 98104140.3
(22) Anmeldetag: 09.03.1998
(51) Int. Cl.: C07D 301/26, C07D 303/08

(54) **Verbessertes Verfahren zur Herstellung von Trifluormethyloxiran**

(30) Priorität: 21.03.1997 DE 19711826
(71) Anmelder: BAYER AG, 51368 Leverkusen (DE)
(72) Erfinder: Rock, Michael-Harold, Dr., 51065 Köln (DE); Lui, Norbert, Dr., 51061 Köln (DE)

(57) **Zusammenfassung**

Bei einem verbesserten Verfahren zur Herstellung von Trifluormethyloxiran wird so verfahren, daß man 3-Chlor-1,1,1-trifluoraceton in Gegenwart eines inerten Lösungsmittels reduziert, danach das Reaktionsgemisch mit einem Protonenspender versetzt, so 3-Chlor-1,1,1-trifluor-2-propanol erhält und dieses durch Einwirkung von wäßriger Lauge unter Eliminierung von Chlorwasserstoff bei Temperaturen unter 70°C cyclisiert.

## Beschreibung

Die vorliegende Erfindung betrifft ein Verfahren zur Herstellung von Trifluormethyloxiran aus 3-Chlor-1,1,1-trifluoraceton.

Trifluormethyloxiran ist ein Zwischenprodukt z.B. für die Synthese antiviraler Mittel (siehe Nucleosides Nucleotides 15, 1315 (1996)) und die Herstellung von Detektoren für hypoxische Turmorzellen (siehe WO 96/04249).

Trifluormethyloxiran kann man bisher beispielsweise aus 1,1,1-Trifluorpropen durch Herstellung des 2-Brom-3-acetats und anschließender Eliminierung mit Natriumhydroxid herstellen (siehe Izv. Akad. Nauk. SSSR Ser. Khim. 1956, 843). Nachteilig ist die Notwendigkeit des Einsatzes von toxischem Quecksilberacetat in ungefähr molaren Mengen.

Trifluormethyloxiran kann auch durch die Reaktion von Diazomethan mit Trifluoracetaldehyd hergestellt werden (siehe J. Org. Chem 25, 102 (1960)). Diese Methode ist wegen der toxischen Eigenschaften und der Explosiongefährlichkeit von Diazomethan für größere Ansätze jedoch völlig ungeeignet.

In J. Am. Chem. Soc. 74, 3022 (1952) ist beschrieben, daß man Trifluormethyloxiran in guten Ausbeuten über zwei Stufen aus 3-Brom-1,1,1-trifluoraceton erhält. Zunächst liefert die Reduktion mit Lithiumaluminiumhydrid 3-Brom-1,1,1-trifluor-2-propanol, aus dem dann mit konzentrierter Natronlauge bei 100°C Bromwasserstoff eliminiert wird. Es wird darauf hingewiesen, daß bei dieser Eliminierung niedrigere Temperaturen, verdünntere Lösungen und längere Kontaktzeiten die unerwünschte Bildung des entsprechenden Glykols begünstigen. Nachteilig aber ist vor allem der Einsatz des nur aufwendig herzustellenden Bromtrifluoracetons, das nur in einer vielstufigen Synthese aus Trifluoressigester zugänglich ist. Der letzte Schritt bei dieser Herstellung von Bromtrifluoraceton ist die Bromierung von 1,1,1-Trifluoraceton in konzentrierter Schwefelsäure (siehe J. Am. Soc. 74, 3902 (1952)). In J. Org. Chem. 60, 41 (1995)) ist die oben erwähnte Eliminierung an enantiomerenreinem 3-Brom-1,1,1-trifluor-2-propanol beschrieben. Es wird zunächst zu eiskalter 41 gew.-%-iger wäßriger Natronlauge gegeben. Die Reaktion wird dann bei 95 - 100°C durchgeführt. Schließlich ist aus JP 06/247953 (zitiert in C.A. 122:9847) die Umsetzung von 3,3,3-Trifluorpropan-1,2-diol mit Sulfurylchlorid zum entsprechenden Chlorhydrin beschrieben, welches mit in Diglyme suspendiertem Natriumhydroxid zum Trifluormethyloxiran cyclisiert wird. Die Ausbeute über beide Stufen beträgt aber nur 8 % d.Th..

Es wurde nun ein Verfahren zur Herstellung von Trifluormethyloxiran gefunden, das dadurch gekennzeichnet ist, daß man 3-Chlor-1,1,1-trifluoraceton in Gegenwart eines inerten Lösungsmittels reduziert, danach das Reaktionsgemisch mit einem Protonenspender versetzt, so 3-Chlor-1,1,1-trifluor-2-propanol erhält und anschließend dieses durch Einwirkung von wäßriger Lauge unter Eliminierung von Chlorwasserstoff bei Temperaturen von unter 70°C cyclisiert.

Die Reduktion kann beispielsweise mit komplexen Hydriden, wie Natriumborhydrid oder Lithiumaluminiumhydrid durchgeführt werden, bevorzugt ist Lithiumaluminiumhydrid. Es ist auch möglich, durch chirale Induktion mit einem chiralen Reduktionsmittel, beispielsweise B-Chlor-diisopinocampheylboran (DIP-Chlorid) zu enantiomerenreinem 3-Chlor-1,1,1-trifluor-2-propanol zu kommen (siehe auch J. Org. Chem. 60, 41 (1995). Solche Reduktionsmittel sind kommerziell erhältlich. Man kann pro Mol 3-Chlor-1,1,1-trifluoraceton beispielsweise 0,8 bis 2 Äquivalente, bevorzugt 1 bis 1,5 Äquivalente, eines komplexen Hydrids oder beispielsweise 0,9 bis 1,5 mol, bevorzugt 0,95 bis 1,05 mol eines chiralen Reduktionsmittels einsetzen.

Die Reduktion wird in Gegenwart eines inerten Lösungsmittels durchgeführt. Beim Einsatz von Lithiumaluminiumhydrid sind z.B. Ether geeignet, wie Diethylether, Diisopropylether und Tetrahydrofuran, beim Einsatz von Natriumborhydrid oder chiralen Reduktionsmitteln beispielsweise Alkohole, wie Methanol und Ethanol oder Ester, wie Ethylacetat.

Man kann beispielsweise pro Mol 3-Chlor-1,1,1-trifluoraceton 100 bis 2000 ml, bevorzugt 200 bis 1000 ml Lösungsmittel einsetzen. Die Temperaturen können dabei z.B. zwischen -80°C und +50°C, vorzugsweise zwischen -30°C und +30°C liegen.

Nach dem Abklingen der Reaktion wird ein Protonenspender zugefügt und dann das Reaktionsgemisch mit üblichen Methoden, beispielsweise durch Extraktion, Destillation und/oder Chromatographie aufgearbeitet.

Als Protonenspender sind beispielsweise Alkohole, Aminoalkohole, Carbonsäuren und Mineralsäuren geeignet, die man vorzugsweise in Form wäßriger Lösungen anwendet. Bevorzugt sind wäßrige Lösungen von Schwefelsäure und Salzsäure, beispielsweise in Konzentrationen zwischen 10 % und der maximal möglichen. Beim Einsatz chiraler Reduktionsmittel ist Aminoethanol als Protonenspender bevorzugt.

Die Menge an Protonenspender umfaßt die genannten und gegebenenfalls vorhandenes Wasser. Die Menge ist nicht kritisch, vorausgesetzt es werden genügend Protonen zur Verfügung gestellt, um das 3-Chlor-1,1,1-trifluor-2-propanol freizusetzen. Die Zugabe des Protonenspenders kann z.B. bei Temperaturen im Bereich -80 bis +50°C, vorzugsweise -30 bis +20°C erfolgen.

Für die Eliminierung geeignete wäßrige Laugen sind z.B. wäßrige Alkali- und Erdalkalilaugen wie Natronlauge, Kalilauge und Bariumhydroxidlösungen. Auch wäßrige Lösungen von Ammoniak kommen in Frage. Bevorzugt sind Natronlauge und Kalilauge. Besonders bevorzugt ist Natronlauge. Geeignete Konzentrationen sind z.B. solche von 10 Gew-% bis zur Sättigungsgrenze. Pro Mol 3-Chlor-1,1,1-trifluor-2-propanol kann man beispielsweise 1 bis 4, vorzugsweise 1,2 bis 3 Äquivalente wäßrige Lauge einsetzen. Die Eliminierung kann beispielsweise bei -10 bis +70°C, vorzugsweise bei 0 bis +50°C durchgeführt werden.

Die Eliminierung kann man beispielsweise so durchgeführen, daß man die gekühlte wäßrige Lauge vorlegt, das 3-Chlor-1,1,1-trifluor-2-propanol zugibt und anschließend auf Raumtemperatur erwärmen läßt. Anschließend kann man das bei Normaldruck bei 38°C übergehende Trifluormethyloxiran einfach aus dem Reaktionsmischung abdestillieren.

Das gesamte erfindungsgemäße Verfahren wird üblicherweise bei Normaldruck durchgeführt. Es ist jedoch auch möglich, unter vermindertem oder erhöhtem Druck zu arbeiten.

Das erfindungsgemäße Verfahrens hat gegenüber dem Stand der Technik eine Reihe von Vorteilen. So sind die Ausbeuten bei beiden Reaktionsschritten sehr gut. Es kann, was insbesondere überraschend ist, bei deutlich niedrigeren Temperaturen durchgeführt werden als den für die Eliminierung von Bromwasserstoff aus dem analogen Bromhydrin angegebenen 95 bis 100°C. Auch ist das Ausgangsmaterial 3-Chlor-1,1,1-trifluoraceton gegenüber denen gemäß des Standes der Technik, insbesondere gegenüber dem entsprechenden Bromaceton, vorteilhaft, weil es gemäß einem eigenen älteren Vorschlag (siehe deutsche Patentanmeldung 19 54 416.9 und entsprechende Auslandsanmeldungen) auf einfache Weise zugänglich ist. Darüber hinaus ist der Einsatz von Chlorverbindungen gegenüber Bromverbindungen günstiger, weil Brom teurer ist als Chlor und die Entsorgung der als Nebenprodukte gebildeten Chloride unproblematischer durchgeführt werden kann als die Entsorgung der entsprechenden Bromide. Häufig kann man aber Bromverbindungen nicht durch Chlorverbindungen ersetzen, weil die Chlorverbindungen im Vergleich zu entsprechenden Bromverbindungen häufig keine ausreichende Reaktivität aufweisen

Die vorliegende Erfindung wird anhand der folgenden Beispielen noch näher beschrieben.

### Beispiele

### Beispiel 1

### Herstellung von 3-Chlor-1,1,1-trifluor-2-propanol

12 g Lithiumaluminiumhydrid wurden unter einer Stickstoffatmosphäre bei 5°C in 300 ml Diethylether vorgelegt. Dazu wurde eine Lösung von 147 g 1-Chlor-3,3,3-trifluoraceton in 150 ml Diethylether getropft. Anschließend wurde auf Raumtemperatur erwärmen gelassen und über Nacht gerührt. Daraufwurde bei 10°C mit 80 ml 0,5 M Schwefelsäure versetzt, gefolgt von der Zugabe von 70 ml 50 gew.-%-iger Schwefelsäure. Die organische Phase wurde abgetrennt, die wäßrige Phase mit Diethylether extrahiert und die vereinigten organischen Phasen über Natriumsulfat getrocknet. Deren Destillation über eine 30 cm-Vigreux-Kolonne lieferte 132 g (89 % d.Th.) 3-Chlor-1,1,1-trifluor-2-propanol.
¹H-NMR (CDCl₃) δ [ppm]: 3,6 - 3,8 (m, 2H); (s, 3H); 4,1 (s, 1H), 4,25 (bs, 1H)
¹⁹F-NMR (CDCl₃) δ [ppm]: -78 (d)

### Beispiel 2

### Herstellung von Trifluormethyloxiran

Zu einer eisgekühlten wäßrigen Natronlauge (38,5 g Natriumhydroxid gelöst in 180 ml Wasser) wurden 52,8 g 3-Chlor-1,1,1-trifluor-2-propanol (erhalten gemäß Beispiel 1) gegeben. Die milchige Reaktionsmischung wurde unter Rühren auf Raumtemperatur erwärmen gelassen. Darauf wurde unter Erwärmen bei 38 bis 39°C übergehendes Trifluormethyloxiran abdestilliert. Es wurden 29,4 g (74 % d.Th.) erhalten.

## Patentansprüche

1. Verfahren zur Herstellung von Trifluormethyloxiran, dadurch gekennzeichnet, daß man 3-Chlor-1,1,1-trifluoraceton in Gegenwart eines inerten Lösungsmittels reduziert, danach das Reaktionsgemisch mit einem Protonenspender versetzt, so 3-Chlor-1,1,1-trifluor-2-propanol erhält, und anschließend dieses durch Einwirkung von wäßriger Lauge unter Eliminierung von Chlorwasserstoff cyclisiert.

2. Verfahren nach Anspruch 1, dadurch gekennzeichnet, daß man als Reduktionsmittel Natriumborhydrid, Lithiumaluminiumhydrid oder B-Chlor-diisopinocampheylboran verwendet.

3. Verfahren nach Ansprüchen 1 und 2, dadurch gekennzeichnet, daß man pro Mol 3-Chlor-1,1,1-trifluoraceton 0,8 bis 2 Äquivalente Natriumborhydrid oder Lithiumaluminiumhydrid oder 0,9 bis 1,5 mol eines chiralen Reduktionsmittels einsetzt.

4. Verfahren nach Ansprüchen 1 bis 3, dadurch gekennzeichnet, daß man die Reduktion bei -80 bis +50°C und in Gegenwart von Ethern, Alkoholen oder Estern durchführt.

5. Verfahren nach Ansprüchen 1 bis 4, dadurch gekennzeichnet, daß man als Protonenspender Alkohole, Aminoalkohole, Carbonsäuren oder Mineralsäuren einsetzt und die Zugabe des Protonenspenders bei -80 bis +50°C durchführt.

6. Verfahren nach Ansprüchen 1 bis 5, dadurch gekennzeichnet, daß man für die wäßrige Eliminierung wäßrige Alkali- und Erdalkalilauge oder wäßrige Lösungen von Ammoniak verwendet.

7. Verfahren nach Ansprüchen 1 bis 6, dadurch gekennzeichnet, daß man bei der Eliminierung pro Mol 3-Chlor-1,1,1-trifluor-2-propanol 1 bis 4 Äquivalente wäßrige Lauge einsetzt und Temperaturen zwischen -10 bis +70°C wählt.

8. Verfahren nach Ansprüchen 1 bis 7, dadurch gekennzeichnet, daß man die Eliminierung durchführt, indem man die gekühlte wäßrige Lauge vorlegt, das 3-Chlor-1,1,1-trifluor-2-propanol zugibt, anschließend auf Raumtemperatur erwärmen läßt und abschließend das Trifluormethyloxiran aus der Reaktionsmischung abdestilliert.
